Europäisches Patentamt

European Patent Office

Office européen des brevets

(11) Publication number: **0 115 083
B1**

# EUROPEAN PATENT SPECIFICATION

(45) Date of publication of patent specification: **26.03.86**

(21) Application number: **83113212.1**

(22) Date of filing: **29.12.83**

(51) Int. Cl.[4]: **C 07 C 43/11,** C 07 C 41/03, C 08 G 65/28

(54) Catalytic process for the preparation of nonionic surfactants.

(30) Priority: **30.12.82 US 454560**

(43) Date of publication of application:
**08.08.84 Bulletin 84/32**

(45) Publication of the grant of the patent:
**26.03.86 Bulletin 86/13**

(84) Designated Contracting States:
**BE DE FR GB IT NL SE**

(56) References cited:
**EP-A-0 026 544
EP-A-0 026 546**

(73) Proprietor: **UNION CARBIDE CORPORATION
39 Old Ridgebury Road
Danbury Connecticut 06817 (US)**

(72) Inventor: **McCain, James Herndon
1987 Parkwood Road
Charleston West Virginia 25314 (US)**

(74) Representative: **Wuesthoff, Franz, Dr.-Ing. et al
Patentanwälte Wuesthoff -v. Pechmann-
Behrens-Goetz Schweigerstrasse 2
D-8000 München 90 (DE)**

Courier Press, Leamington Spa, England.

## 0 115 083

## Description

### Technical Field

This invention relates to the preparation of improved nonionic surface active agents and, more particularly, to a process for the oxyalkylation of certain reactive hydrogen compounds, i.e. compounds such as alcohols, to prepare nonionic surface active agents having narrow molecular weight distribution and low pour points.

### Background Art

Low molecular weight condensation products of an alkylene oxide, particularly ethylene oxide, or mixtures of alkylene oxides such as ethylene and propylene oxide with an alcohol are well known and for a long time have been prepared commercially for use in detergents, cleansing agents, dry cleaning materials, wetting and emulsifying agents and the like. The products are conventionally produced by reacting a reactive hydrogen compound such as an alcohol with the alkylene oxide in the presence of a strongly alkaline or an acidic catalyst. Such procedures result in the production of a mixture of relatively low molecular weight (up to about 5000) condensation product species containing a number of alcohol derivatives with different molecular proportions of alkoxylate. Thus, the reaction products generally obtained are, in reality, a mixture of derivatives of the alcohol moiety containing different molecular proportions of alkylene oxide units and a wide range of molecular weights as well as certain proportions of unreacted alcohol. Moreover, as is well known, the conventional designation of the number of alkylene oxide units present per molecule of an alcohol alkoxylate is of the average number of alkylene oxide units per molecule and substantial proportions of alcohol alkoxylates are present which have a greater and a lesser number of alkylene oxide units present than the actual average value would indicate.

It is generally desirable to restrict the molecular distribution of the mixture to adjacent analogues of the desired product insofar as possible, since, as is well known, the number of moles of alkylene oxide in the reaction product is a major factor in the properties of such product, but this is quite difficult to control. Acidic catalysts tend to give a narrower molecular distribution than alkaline catalysts, but also contribute to the formation of undesired by-products. Thus, alkaline catalysts which are typically a strong base such as alkali metal hydroxides or alkali alcoholates, prepared as disclosed in the article entitled "Alkoxides, Metal:, in Kirk-Othmer, Encyclopedia of Chemical Technology, John Wiley & Sons (3d ed. 1978), are generally used as the more efficient type of catalyst but the molecular distribution of the resulting products are more diffuse, containing a greater proportion of lower and higher molecular weight species and smaller amounts of the species with the desired number of moles of alkylene oxide. For example, an 8-mole ethylene oxide (EO) adduct of 1-dodecanol will contain not only the 8-mole EO adduct species but also lower mole adducts and higher mole adducts. Lower mole adducts in the product mixture will range down to the 1 mole adduct and higher adducts will extend up to 14 or 15 and beyond. The molecular weight distribution is a measure of the relative amounts of the various adducts in the product mixture and can be represented in the form of a generally bell-shaped curve where the amount of each adduct species is plotted versus the number of moles of epoxide in the species or of a description of the relative amount of each individual adduct. In terms of the bell-shaped curve, a narrower distribution would give a sharper curve, higher at the middle and lower at the ends. A broader distribution curve would be lower at the middle portion of the range and higher at the ends.

Heretofore, several methods have been suggested for providing reaction products of an active hydrogen compound and epoxides which have a narrow range of molecular weights and molecular distribution of the epoxide units, or which reduce or eliminate the production of undesirable poly (alkylene glycol) and cyclic and straight chain ether by-products. For example, in U.S. Patent 4,112,231 it is disclosed that the use of certain neutral inorganic fluoborate and perchlorate salts will catalyze the reaction of epoxides with active hydrogen compounds to give products having a narrower molecular distribution, i.e., a more limited range of molecular species and a larger proportion of desired species, in U.S. Patent No. 3,682,849 improved ethoxylated derivatives of $C_{11}$—$C_{18}$ alcohols are prepared by removing unreacted alcohol and lower ethoxylates from the conventionally produced ethoxylate mixture using vapor phase separation techniques; in U.S. Patent 2,870,220 two-stage process is disclosed for preparing monoalkyl ethers of ethylene glycol and polyethylene glycols of more restricted molecular weight range wherein an alkanol and ethylene oxide are reacted in the presence of an acidic catalyst during the first stage and then in the second-stage, after removal of acid catalyst and unreacted alkanol, reacting the mixture with ethylene oxide in the presence of an alkali metal alcoholate of the initial alkanol; and in U.K. Patent No. 1,501,327 is disclosed a method of preparing mono- and poly-glycol by-products which involves heating a reaction mixture containing an alkylene oxide and an alcohol in the presence of a catalyst containing alkali or alkaline earth cations wherein some or all of the catalyst is an anhydrous high boiling liquid residue prepared by concentrating the liquid residue from the same or different etherification process after removal of the glycol ether product from the reaction mixture. However, none of these processes or special catalysts are completely satisfactory in that they may require multi-stage procedures or special acid-resistant equipment, may form undesirable by-products or simply do not provide sufficient control over the molecular weight distribution.

Recently, several patents were issued which are also concerned with the preparation and advantages

2

of nonionic surfactant products having a narrower molecular weight distribution. For example, in U.S. Patent 4,239,917 is disclosed the use of certain basic barium materials, namely barium oxide, hydrated barium oxides, and barium metal, as catalysts in the preparation of reaction products of alcohols and ethylene oxide which have a narrow, high mole adduct distribution while providing low levels of undesirable by-products and unreacted free alcohol. The significance of the molecular weight distribution of the products produced during the oxyalkylation reaction is discussed at length by patentee and the differences in the molecular weight distribution of reacton products prepared with conventional alkali metal catalysts such as sodium hydroxide and those prepared using a barium catalyst of the invention is shown by graphical representations. This patent also shows that other alkaline earth metal materials, such as calcium hydroxide, magnesium oxide, and strontium hydroxide, were ineffective as catalysts for the oxyalkylation reaction. Thus patentee demonstrates that significant differences exist not only between the catalytic effect of the barium oxide, hydrated barium oxide and barium metal catalysts of the invention and conventional alkali metal hydroxide catalysts but in the catalytic effectiveness of the various alkaline earth metals as well.

U.S. Patent 4,210,764 is concerned with the molecular weight distribution of products prepared by oxyalkylation of alcohols using conventional alkaline catalysts and is directed to employing barium containing catalysts such as disclosed in U.S. Patent No. 4,239,917 in conjunction with various phenols. Patentees suggest that the use of various phenols as a promoter overcomes an induction period problem found when the basic barium materials are used by themselves. U.S. Patent 4,223,164, which is also concerned with the molecular weight distribution of products prepared by the oxyalkylation of alcohols, discloses that the use of certain basic strontium salts when used in conjunction with various phenols produces surfactants with narrow molecular weight distributions.

In co-pending applications Serial Nos. 079,497 and 079,539 both filed September 27, 1979 there are described oxyalkylation catalysts prepared from barium metal and barium oxide, respectively. These oxyalkylation catalysts, when used in the oxyalkylation process described in co-pending application Serial No. 275,031, produce nonionic surfactants with a narrow molecular weight range. The oxyalkylation catalyst of Serial Nos. 079,497 and 079,539 are prepared by reacting barium metal or barium oxide with a 1—4 carbon alkanol and subsequently displacing the 1—4 carbon alkanol with a surfactant alkanol by distillation to remove the lower alkanol in equilibrium with the lower alkoxide. The reaction of barium metal or barium oxide with a lower alkanol is generally characterized by a vigorous reaction indicated by the evolution of hydrogen gas in the case of barium metal or the exothermic dissolution of the barium oxide. However, it was found that barium hydroxide does not appear to react with a 1—4 carbon alkanol to form an alkoxide. This is not surprising in light of the fact that a hydroxide ion is generally a weaker base than an alkoxide ion. See Morrison and Boyd, *Organic Chemistry* p. 526, Allyn and Bacon, Inc. (3d ed. 1973).

Disclosure of the Invention

In accordance with the present invention there is provided a process for the preparation of nonionic surfactants having a narrow molecular weight distribution which comprises reacting a reactive hydrogen compound selected from the group of higher monohydric alcohols having from 8 to 20 carbon atoms and difunctional propylene oxide polymers having an average molecular weight from 1000 g/mol to 5000 g/mol with an alkylene oxide having 2 to 4 carbon atoms at a temperature at which the reaction proceeds in the presence of at least a catalytic amount of the reaction product of barium hydroxide or barium hydroxide hydrate and a lower monohydric aliphatic alcohol having 1 to 4 carbon atoms.

It has been discovered that, although barium hydroxide and barium hydroxide hydrate often do not appear to react with a 1—4 carbon alkanol to form a lower alkoxide as do barium metal and barium oxide, barium hydroxide and barium hydroxide hydrate, when treated with a 1—4 carbon alkanol form a species that is catalytically active in oxyalkylating a surfactant alkanol. It has also been discovered that the basic salts of barium herein described not only catalyze the reaction of the active hydrogen compound and alkylene oxide but also favor the formation of products having a narrower molecular distribution, i.e., a more limited range of molecular species and a larger proportion of the desired species in the reaction product than is prepared with conventional alkali metal catalysts such as potassium hydroxide. Moreover, the process of the invention can be readily carried out in a single stage with substantially no delay or induction period, and without the need for special acid-resistant equipment. The products thereby have been found, in general, to exhibit improved properties such as lower pour points and to contain only small amounts of undesired poly(alkylene glycol) and ether by products.

The catalytically active species of the present invention is formed by reacting barium hydroxide or barium hydroxide hydrate with a lower monohydric alcohol having from 1 to 4 carbon atoms. Suitable lower monohydric alcohols include methanol, ethanol, n-propanol, iso-propanol, n-butanol, sec-butanol, iso-butanol, and tertiary butanol. The amount of lower monohydric alcohol used is not narrowly critical and can range from 70% to 1% by weight of the barium hydroxide or barium hydroxide hydrate. However, because removal of the lower monohydric alcohol after addition of the higher monohydric alcohol or difunctional propylene oxide polymer is preferred, only so much lower monohydric alcohol as is necessary to form a catalytically active species is preferably added. If the lower monohydric alcohol is not removed from the reaction mixture, then an amount of lower alcohol less than 3% by weight is preferably added to obtain a nonionic surfactant with acceptable surfactant properties and a narrow molecular weight distribution.

The amount of catalyst used in accordance with the invention is not narrowly critical and a catalytic effect has been noted with only a small amount thereof being present. The catalytically active species may be soluble in reaction mixture or may be absorbed onto the surface of any unreacted barium hydroxide or barium hydroxide hydrate. In general, the catalyst concentration can vary from 0.001 percent by weight to 10 percent by weight of barium hydroxide or barium hydroxide hydrate based on the weight of active higher monohydric alcohol or difunctional propylene oxide polymer hydrogen compound. Concentrations of alkaline earth metal within the range from 0.05 percent to 5.0 percent by weight of active hydrogen compound are usually preferred. The reaction rate, however, is dependent on both the temperature and the catalyst concentration and to achieve a given rate, more catalyst is required at a low temperature than at a high temperature.

In accordance with the present invention there is provided a process for the production and use of a barium species from barium hydroxide or barium hydroxide hydrate that will produce a nonionic surfactant with a narrow molecular weight distribution. In general, barium hydroxide or a hydrate thereof, is mixed with a lower monohydric alcohol having 1 to 4 carbon atoms to form a barium species that is catalytically active in the oxyalkylation of a higher monohydric alcohol or a difunctional propylene oxide polymer. A higher monohydric alcohol having from 8 to 20 carbon atoms or a difunctional propylene oxide polymer with an average molecular weight between 1000 g/mol to 5000 g/mol is then added to the barium hydroxide-lower alcohol mixture. The lower alcohol is then preferably removed, preferably by distillation, but such a distillation is not necessary to produce a nonionic surfactant with a narrow molecular weight distribution. Removal of the lower alcohol may be conducted by any separation means that retains the catalytic activity of the basic barium species. Preferably, distillation techniques are used, but other methods such as column chromatography, fractional freezing and the like may also be employed. The lower alcohol is removed from the reaction mixture by distillation at a temperature and pressure whereby, for example, the lower alcohol is vaporized and the higher alcohol is not, the temperature being dependent on the pressure and types of higher and lower alcohols that are used in the reactions.

The reaction may then be conducted in a conventional manner, that is, an alkylene oxide is added to a reactor containing a reactive hydrogen compound and a catalyst until the desired number of moles have been reacted therewith, and the product is removed from the reactor and neutralized with acid. The reaction may be conducted in the presence of a solvent, but usually a solvent is not employed.

The temperature at which the reaction proceeds is not narrowly critical and generally products can be made at a reasonable rate of reaction and without decomposition of the reactants or reaction products at a temperature between 50°C and 270°C, with a temperature between 100°C and 200°C being generally preferred. While the pressure of the reaction is not narrowly critical when low-boiling epoxides, such as ethylene oxide and propylene oxide are employed, a pressurized reactor is preferably used.

The product may be neutralized with any acid that will convert the catalyst to a neutral salt, as for example, acetic acid, carbon dioxide, sulfuric acid, and phosphoric acid.

The reactive hydrogen compound of the present invention is generally a higher aliphatic or aromatic monohydric alcohol or polyether polyol. Higher monohydric alcohols which are suitable for use in the practice of the invention are primary and secondary aliphatic alcohols which are straight or branched chain and have from about eight to about twenty carbon atoms. Exemplary of such suitable primary straight chain aliphatic alcohols are n-octanol, n-nonanol, n-decanol, n-dodecanol, n-tridecanol, n-tetradecanol, n-pentadecanol, n-hexadecanol, n-heptadecanol, n-octadenanol, n-nonadecanol and eicosanol, and of such branched chain alcohols are 2-methyl-1-nonanol, 2-methyl-1-undecanol, 2-methyl-1-dodecanol and 2-methyl-1-tetradecanol. Exemplary secondary alcohols include 2-octanol, 2-dodecanol, 4-tetradecanol and 6-heptadecanol. Mixtures of such alcohols including commercially available alcohols which normally comprise mixtures of alcohol are also suitable. Particularly preferred are linear and branched primary alcohols and alcohol mixtures such as are produced by the "Oxo" reaction of normal $C_7$—$C_{19}$ olefins.

Also suitable are cycloaliphatic monohydric alcohols, including, for example, cyclohexanol, cyclopentanol, cycloheptanol, cyclopropanol and cyclooctanol, as well as phenyl-substituted monohydric alcohols such as benzyl alcohol, phenylethyl alcohol, and phenylpropyl alcohol. Suitable phenols include for example phenol, and alkyl phenols such as p-methylphenol, p-ethyl phenol, p-butyl phenol, p-heptylphenol, p-nonyl phenol, dinonyl phenol and p-decylphenol. The aromatic radicals may contain other conventional substituents such as halide atoms.

Other suitable alcohols are those derived by hydrogenation of natural fats and oils, such as CO and TA alcohols, trademark of and sold by Proctor and Gamble Co., such as CO-1214N alcohol, CO-1618 alcohol, and TA 1618 alcohol, and ADOL alcohols, trademark of and sold by Ashland Oil Co., such as ADOL 54 alcohol, ADOL 61 alcohol, ADOL 64 alcohol, ADOL 60 alcohol and ADOL 66 alcohol. Alcohols produced by Ziegler chemistry can also be alkoxylated. Examples of these alcohols are ALFOL alcohols, trademarks of and sold by Continental Oil Co., such as ALFOL 1012 alcohol, ALFOL 1214 alcohol, ALFOL 1412 alcohol, ALFOL 1618 alcohol, ALFOL 1620 alcohol; and EPAL alcohols, trademark of and sold by Ethyl Chemical Co., such as EPAL 1012 alcohol, EPAL 1214 alcohol, EPAL 1418 alcohol. The invention is extremely useful for oxo alcohols (hydroformulation) produced from olefins. Examples of such alcohols are NEODOL alcohol, trademark of and sold by Shell Oil Co., such as CEODOL 23 alcohol, NEODOL 25 alcohol, NEODOL 1418 alcohol; TERGITOL-L, trademark of Union Carbide Corp., such as TERGITOL-L 125 alcohol; LIAL alcohols, trademark of and sold by Liquidhimica Co. such as LIAL 125; and isodecyl and tridecyl alcohols, sold by Exxon Corp., such as isodecyl alcohol and tridecyl alcohol, Guebet alcohols can also be ethoxylated. Representative examples of these alcohols are STANDAMUL alcohols, trademarks of and sold by Henkel Chemical Co., such as STANDAMUL GT-12 alcohol, STANDAMUL GT-16 alcohol, STANDAMUL GT-20 alcohol, STANDAMUL GT-1620 alcohol. Secondary alcohols can also be used, such as TERGITOL 15 alcohol, trademark of and sold by Union Carbide Corp.

Also suitable are difunctional propylene oxide polymers having a molecular weight of 1000 to 5000, and preferably 1700 to 4100. The propylene oxide polymers having a molecular weight of 1000 to 5000 contain from 17 to 86 oxypropylene units in the molecular. These compounds are well known, being generally obtained by polymerization of propylene oxide or by the addition of propylene oxide to lower molecular compounds with 2 to 6 carbon atoms containing at least 2 reactive hydrogen atoms. The amount of higher alcohol or polyol mixed with the lower alcohol-barium hydroxide reaction mixture should be sufficient to convert all of the reaction product of the barium salt and the lower alcohol to a soluble higher alcohol basic salt thereof; i.e., a higher alcohol alkoxide and preferably at least 10 percent greater than that stoichiometrically required, calculated on the assumption that the basic barium species is the barium alkoxide of the lower alcohol.

Alkylene oxides suitable for use in accordance with the invention have from 2 to 4 carbon atoms and include, for example, ethylene oxide, 1,2-propylene oxide, 1,2-butylene oxide, and mixtures thereof. The number of moles of alkylene oxides employed according to the present invention may vary widely depending on the reactive hydrogen compound to be adducted and the particular application for which the surface active agent is to be employed. In general 2 to 60 and even more moles of alkylene oxide per mole of reactive hydrogen compound may be used. Insofar as propylene oxide and/or butylene oxide are used in combination with ethylene oxide, the molar ratio to ethylene oxide to propylene — or butylene oxide may be from 1:0.03 to 1:1.

The products prepared by the reaction of a reactive higher monohydric alcohol or propylene oxide polymer hydrogen compound and an alkylene oxide in accordance with the practice of the invention are, in general, a mixture of relatively low molecular weight alkoxylates (molecular weight up to about 5000) which are efficient nonionic surfactants wherein the molecular weight distribution of the alkoxylate species in the mixture is narrower than is prepared using conventional alkali metal catalysts such as potassium hydroxide. Thus, products are prepared having a greater proportion of the desired alkoxylate species, that is, having a greater proportion of products with the desired number of alkylene oxide groups. Moreover, products prepared in accordance with the invention generally have a lesser amount of unreacted alcohol and undesirable poly(alkylene glycol) by-products as well as exhibiting improved properties such as a lower pour points than products prepared by the reaction of comparable amounts of alkylene oxide and alcohol reactants in the presence of conventional alkaline catalysts.

The invention will become more clear when considered together with the following examples which are set forth to illustrate but not limit the present invention. Unless otherwise indicated, all parts and percentages are by weight.

## EXAMPLES
### Examples 1 and 2
Example 1

To a quantity of methanol (400 cm$^3$) was added barium hydroxide monohydrate (13.73 g., 0.073 moles), and the mixture was heated at reflux at atmospheric pressure for 2 hours. LIAL-125 alcohol, a $C_{12}$—$C_{15}$ mixture of primary alcohols, 40% normal, 60% branched, available for Liquichemica Italia (500g) was added and the methanol was removed by heating to 90°C and 6,65 mbar (5 mm pressure). The mixture of barium salt and LIAL-125 alcohol was transferred to a 7,57 l (2-gallon) stirred, stainless steel autoclave. The autoclave was then purged three times with nitrogen, heated to 140°C, and pressurized to 1,38 bar (20 psig) with nitrogen. Ethylene oxide from a calibrated tank was added until the pressure reached 4,14 bar (60 psig). Thereafter, as ethylene oxide reacted and the pressure fell, more ethylene oxide was added to maintain 4,14 bar (60 psig). When the desired amount of ethylene oxide had reacted, the reactor was cooled and the product neutralized with acid to pH 7. In this example, 29 minutes were required to react 775 ml of ethylene oxide. The product, a poly(ethylene oxide) adduct of LIAL-125 alcohol, had a molecular weight of 554 and a cloud point (1 percent aqueous) of 47.5°C.

### Comparative Example 2

The procedure of Example 1 was repeated except barium hydroxide monohydrate (13.83, .074 moles) was added directly to LIAL-125 alcohol (500 g) with stirring. A total of 112 minutes were required to react 775 ml of ethylene oxide. The product had a molecular weight of 500 and a cloud point (one percent aqueous) of less than 25°C.

A comparison of Examples 1 and 2 shows that the use of a lower monohydric alcohol substantially shortens the time required to produce a given amount of nonionic surfactant through the oxyalkylation of a higher monohydric alcohol.

### Examples 3—5

#### Example 3

This shows the preferred embodiment with another alcohol. Barium hydroxide monohydrate (16.47 g, 0.0876 moles) was refluxed with methanol (400 cm³) by the procedure of Example 1, then 2-ethylhexanol (600 g) was added and the methanol removed in vacuo. The mixture of barium salt and 2-ethylhexanol was charged to the autoclave of Example 1 and reacted under the conditions of Example 1 with 1535 cm³ of ethylene oxide. The reaction was complete after 43 minutes. The product was a 2-ethylhexanol-started poly (ethylene oxide) with a molecular weight of 417.8 and a cloud point (1% aqueous) of 66°C.

Gas chromatographic analysis of the trimethylsilyl derivative of the product (Table I) showed a narrow molecular weight distribution.

#### Comparative Example 4

Barium hydroxide monohydrate (16.47 g, 0.0876 moles) was stirred into 2-ethylhexanol (600 g) and the mixture was charged to the autoclave of Example 1. At the condition of Example 2, 217 minutes were required to react 1545 cm³ of ethylene oxide. The product, a 2-ethylhexanol-started poly(ethylene oxide), had a molecular weight of 437 and a cloud point (1% aqueous) of 64°C. Gas chromatographic analysis of the trimethylsilyl derivative of the product (Table I) showed a narrow molecular weight distribution. A comparison of the reaction times of examples 3 and 4 shows that the use of a lower alcohol substantially improves the catalytic activity of a barium catalyst.

#### Comparative Example 5

Sodium hydroxide, (3.01 g, 0.0750 moles) and 2-ethylhexanol, 500 g, were stirred and heated at 70°C under vacuum. Reaction in the autoclave and by the procedure of Example 1 required 60 minutes for the reaction of 1260 cm³ of ethylene oxide. The product, a 2-ethylhexanol-started poly (ethylene oxide) had a molecular weight of 466 and a cloud point (1 percent aqueous) of 72°C. Gas chromatographic analysis of the trimethylsilyl derivative of the product (Table I) showed a broad molecular weight distribution.

TABLE I

Area Percent of Alcohol and Individual Ethylene Oxide Adducts of 2-ethylhexanol in products from various catalysts

| Example | Area Percent by Gas Chromatography | | |
| --- | --- | --- | --- |
| | 3 | 4 | 5 |
| Unreacted Alcohol | 7.9 | 9.1 | 17.2 |
| Alcohol $E_1$* | 2.4 | 2..4 | 5.3 |
| $E_2$ | 3.4 | 2.4 | 6.0 |
| $E_3$ | 6.8 | 5.4 | 7.1 |
| $E_4$ | 8.8 | 7.0 | 7.0 |
| $E_5$ | 11.0 | 8.7 | 7.7 |
| $E_6$ | 12.8 | 10.5 | 7.7 |
| $E_7$ | 12.6 | 11.2 | 7.8 |
| $E_8$ | 11.0 | 11.3 | 7.3 |
| $E_9$ | 8.7 | 9.8 | 6.7 |
| $E_{10}$ | 6.5 | 7.9 | 5.6 |
| $E_{11}$ | 4.3 | 5.7 | 4.7 |
| $E_{12}$ | 2.6 | 3.9 | 4.2 |
| $E_{13}$ | 1.3 | 2.5 | 3.6 |
| $E_{14}$ | 0.5 | 1.4 | 3.1 |
| $E_{15}$ | | 0.6 | 2.6 |
| $E_{16}$ | | 0.2 | 2.2 |
| $E_{17}$ | | | 1.7 |

*$E_1$ is the 1 mole ethylene oxide adduct, $E_2$ is the 2 mole ethylene oxide adduct, etc.

Table I illustrates that the use of a lower alcohol leads to a narrower molecular weight distribution than is obtained by the sodium hydroxide catalyst of the prior art.

## EXAMPLES 6—8

Example 6

Barium hydroxide monohydrate (52.4 g, 0.279 moles) and methanol (1200 cm$^3$) was heated at reflux with stirring for 4 hours. After filtration through No. 1 filter paper a 396 g portion of the solution was added to 600 g of CO-1214 alcohol, a $C_{12}$—$C_{14}$ primary alcohol mixture available from Proctor and Gamble. The methanol was removed at 6.65 mbar (5 mm) and 90°C and the resulting product was placed in the autoclave of Example 1. At the conditions of Example 1, ethylene oxide, 990 cm$^3$ reacted in 31.5 minutes. The product, a nonionic surfactant, had a cloud point (1% aqueous) of 49°C.

Example 7

Methanol (6.02 g), barium hydroxide monohydrate (17.8 g, 0.095 moles) and CO-1214 alcohol (600 g) were stirred together. Reaction with ethylene oxide (1040 cm$^3$) under the conditions of Example 1 required 91 minutes.

### Comparative Example 8

Barium hydroxide monohydrate (17.8 g, .095 moles) was stirred with CO-1214 alcohol (600 g) in the absence of a lower alcohol. The mixture was added to the autoclave of Example 1. Under conditions of Example 1 ethylene oxide (965 cm$^3$) reacted in 138 minutes. The product was a nonionic surfactant with a cloud point (1% aqueous) of 38.5°C.

The foregoing three examples show that the shortest reaction time is obtained when the lower monohydric alcohol is removed, but that use and non-removal of a lower monohydric alcohol is still preferable to the failure to use a lower monohydric alcohol.

### EXAMPLES 9—12

### Example 9

This example illustrates the use of ethanol. Barium hydroxide monohydrate (16.4 g, .087 moles) and ethanol (400 cm$^3$) were refluxed 2 hours. After cooling to 40°C 1-dodecanol (600 g) was added. The whole was heated to 90°C at 6,65 mbar (5 mm) while ethanol was removed. Under the condition of Example 1, 63 minutes were required to react the mixture with 1075 cm$^3$ of ethylene oxide. The product, a nonionic surfactant, had a molecular weight of 473 and a cloud point (1% aqueous) of 56°C. Gas chromatographic analysis of the trimethylsilyl derivative of the product (Table II) showed a narrow molecular weight distribution.

### Example 10

This illustrates the use of n-propanol. The procedure of Example 8 was repeated using n-propanol instead of ethanol. Seventy-two minutes were required to react 1055 cm$^3$ of ethylene oxide. The product, a nonionic surfactant, had a molecular weight of 494 g and a cloud point (one percent aqueous) of 59.5°C. Gas chromatographic analysis of the trimethylsilyl derivative of product (Table II) showed a narrow molecular weight distribution.

### Comparative Example 11

This is a control for Examples 9 and 10 showing the molecular weight distribution with a conventional catalyst and 1-dodecanol. Sodium hydroxide (3.59 g, 0.090 moles) was added to 1-dodecanol (701 g) and the mixture was heated with stirring to 110°C at 6,65 mbar (5 mm pressure). A portion (606 g) was charged to the autoclave of Example 1 and reacted with ethylene oxide under the conditions of Example 1. 63 minutes were required to react 1080 cm$^3$ of ethylene oxide. The product had a molecular weight of 495 g and a cloud point (1% aqueous) of 55°C. Gas chromatographic analysis of the trimethylsilyl derivative of the product (Table II) showed a broad molecular weight distribution.

### Comparative Example 12

This is a comparative example showing barium hydroxide without lower alcohol. Barium hydroxide (15.15 g, 0.080 mole) and 1-dodecanol (550 g) were mixed and then heated together with stirring to 110°C at 6,65 mbar (5 mm pressure). To the autoclave of Example 1, a 534 g portion of the mixture was added. At the conditions of Example 1, 135 minutes were required to react 950 cm$^3$ of ethylene oxide. The product had a molecular weight of 480 and a cloud point (1% aqueous) of 50°C. Gas chromatographic analyses of the acetate derivative of the product is shown in Table II.

8

TABLE II
Area percent of alcohol and individual ethylene oxide adducts of 1-dodecanol in products from various catalysts

| Example | Area Percent by Gas Chromatography | | | |
|---|---|---|---|---|
| | 9 | 10 | 11 | 12 |
| Unreacted Alcohol | 2.2 | 2.2 | 5.4 | 2.6 |
| Alcohol $E_1$* | 1.7 | 1.5 | 3.6 | 3.3 |
| $E_2$ | 2.9 | 3.3 | 4.5 | 3.7 |
| $E_3$ | 5.4 | 6.0 | 6.0 | 6.5 |
| $E_4$ | 8.3 | 9.1 | 6.9 | 9.8 |
| $E_5$ | 11.5 | 12.7 | 7.7 | 13.0 |
| $E_6$ | 13.9 | 14.1 | 8.3 | 14.7 |
| $E_7$ | 14.6 | 14.5 | 8.8 | 14.3 |
| $E_8$ | 13.1 | 12.6 | 9.0 | 12.2 |
| $E_9$ | 10.4 | 9.8 | 8.6 | 9.1 |
| $E_{10}$ | 7.3 | 6.9 | 7.7 | 6.0 |
| $E_{11}$ | 4.6 | 4.2 | 6.6 | 3.4 |
| $E_{12}$ | 2.6 | 2.0 | 5.4 | 1.7 |
| $E_{13}$ | 1.2 | 0.8 | 4.2 | 0.6 |
| $E_{14}$ | 0.4 | 0.2 | 3.2 | 0.1 |
| $E_{15}$ | | | 2.3 | |
| $E_{16}$ | | | 1.6 | |
| $E_{17}$ | | | 0.1 | |

*$E_1$ is the 1 mole ethylene oxide adduct, $E_2$ is the 2 mole ethylene oxide adduct, etc.

The foregoing examples and Table II illustrate the advantages of using various lower monohydric alcohols to obtain reaction rates faster than when barium hydroxide is used alone as a catalyst and to obtain molecular weight ranges narrower than when sodium hydroxide is used as a catalyst.

**Claims**

1. A process for the preparation of nonionic surfactants having a narrow molecular weight distribution which comprises reacting a reactive hydrogen compound selected from the group of higher monohydric alcohols having from 8 to 20 carbon atoms or difunctional propylene oxide polymers having an average molecular weight from 1000 to 5000 with ethylene oxide at a temperature at which the reaction proceeds in the presence of a catalytic amount of the reaction product of barium hydroxide or barium hydroxide hydrates and a lower monohydric aliphatic alcohol having from 1 to 4 carbon atoms.

2. The process of claim 1 wherein the temperature at which the reaction proceeds is from 50°C to 270°C.

3. The process of claim 1 or 2 wherein the reactive hydrogen compound is an aliphatic monohydric alcohol having from 8 to 20 carbon atoms.

4. The process of claim 1 to 3 wherein the lower monohydric alcohol is removed from the reaction mixture by distillation.

5. The process of claim 1 to 4 wherein the amount of the reactive hydrogen compound added to the reaction

**0 115 083**

mixture is sufficient to convert all of the reaction product of barium hydroxide or barium hydroxide hydrates and lower alcohol basic salts to a higher alcohol basic salt.

**Patentansprüche**

1. Verfahren zur Herstellung nicht-ionischer Tenside mit enger Molekulargewichtsverteilung durch Umsetzung einer reaktiven Wasserstoffatome enthaltenden Verbindung in Form höherer einwertiger Alkohole mit 8 bis 20 Kohlenstoffatomen oder bifunktionellen Propylenoxidpolymeren mit einem mittleren Molekulargewicht von 1000 bis 5000 mit Ethylenoxid bei einer Temperatur, bei der die Reaktion erfolgt, in Gegenwart einer katalytischen Menge eines Reaktionsprodukts von Bariumhydroxid oder Bariumhydroxidhydraten und einem niederen einwertigen aliphatischen Alkohol mit 1 bis 4 Kohlenstoffatomen.

2. Verfahren nach Anspruch 1, wobei die Reaktion bei einer Temperatur zwischen 50 und 270°C durchgeführt wird.

3. Verfahren nach Anspruch 1 oder 2, worin die reaktive Wasserstoffatome enthaltende Verbindung ein aliphatischer einwertiger Alkohol mit 8 bis 20 Kohlenstoffatomen ist.

4. Verfahren nach Anspruch 1 bis 3, worin der niedere einwertige Alkohol aus dem Reaktionsgemisch abdestilliert wird.

5. Verfahren nach Anspruch 1 bis 4, worin die reaktive Wasserstoffatome enthaltende Verbindung, die in das Reaktionsgemisch eingebracht wird, ausreicht, um das gesamte Reaktionsprodukt von Bariumhydroxid oder Bariumhydroxidhydraten und basischen Salzen des niederen Alkohols in ein basisches Salz eines höheren Alkohols umzuwandeln.

**Revendications**

1. Procédé de préparation de surfactants non ioniques à distribution étroite des poids moléculaires, qui consiste à faire réagir un composé à hydrogène réactif choisi dans le groupe des alcools monohydroxyliques supérieurs ayant 8 à 20 atomes de carbone ou des polymères difonctionnels d'oxyde de propylène ayant un poids moléculaire moyen de 1000 à 5000 avec l'oxyde d'éthylène à une température à laquelle la réaction s'effectue en présence d'une quantité catalytique du produit de réaction d'hydroxyde de baryum ou des hydrates d'hydroxyde de baryum et d'un alcool aliphatique monohydroxylique inférieur ayant 1 à 4 atomes de carbone.

2. Procédé suivant la revendication 1, dans lequel la température à laquelle la réaction s'effectue va de 50 à 270°C.

3. Procédé suivant la revendication 1 ou 2, dans lequel le composé à hydrogène réactif est un alcool monohydroxylique aliphatique ayant 8 à 20 atomes de carbone.

4. Procédé suivant les revendications 1 à 3, dans lequel l'alcool monohydroxylique inférieur est éliminé du mélange réactionnel par distillation.

5. Procédé suivant les revendications 1 à 4, dans lequel la quantité de composé à hydrogène réactif ajoutée au mélange réactionnel est suffisante pour transformer la totalité du produit de réaction de l'hydroxyde de baryum ou des hydrates d'hydroxyde de baryum et des sels basiques d'alcools inférieurs en un sel basique d'alcool supérieur.